# EUROPEAN PATENT SPECIFICATION

(11) **EP 1 702 639 B1**
(45) Date of publication and mention of the grant of the patent: **18.02.2009**
(21) Application number: 06013138.0
(22) Date of filing: 24.05.2000
(51) Int. Cl.: A61M 15/00

(54) **Apparatus for dispensing metered amount of aerosolized medication**
Gerät zum Abgeben von abgemessenen Mengen von Medikamente beinhaltendem Aerosol
Appareil de distribution de doses de médicament sous forme d'aérosol

(30) Priority: 28.05.1999 US 136518 P; 24.04.2000 US 556262
(43) Date of publication of application: 20.09.2006
(62) Divisional of application: 00936239.3
(73) Proprietor: Nektar Therapeutics, San Carlos, CA 94070 (US)
(72) Inventor: Schuler, Carlos, Cupertino, CA 95014 (US); Paboojian, Steve, Menlo Park, CA 94025 (US); Bakshi, Aneesh, K., Belmont, CA 94002 (US); Tuttle, Derrick, San Mateo, CA 94401 (US)
(74) Representative: Vossius & Partner

(56) References cited:
- EP-A- 0 692 434
- WO-A-99/25484
- WO-A-99/26004
- US-A- 4 114 615
- US-A- 5 875 776

## Description

### BACKGROUND OF THE INVENTION

The present invention relates generally to the field of drug delivery, and in particular to the pulmonary delivery of drugs. More specifically, the invention relates to the aerosolization of pharmaceutical formulations for inhalation by a patient.

Effective drug delivery to a patient is a critical aspect of any successful drug therapy. Various routes of delivery exist, and each has its own advantages and disadvantages. Oral drug delivery of pills, capsules, elixirs, and the like, is perhaps the most convenient method, but many drugs are degraded in the digestive tract before they can be absorbed. Such degradation is a particular problem with modern protein drugs which are rapidly degraded by proteolytic enzymes in the digestive tract. Subcutaneous injection is frequently an effective route for systemic drug delivery, including the delivery of proteins, but enjoys a low patient acceptance. Since injection of drugs, such as insulin, one or more times a day can frequently be a source of poor patient compliance, a variety of alternative routes of administration have also been developed, including transdermal , intranasal, intrarectal, intravaginal, and pulmonary delivery.

Of particular interest to the present invention, pulmonary drug delivery relies on inhalation of a drug dispersion or aerosol by the patient so that the active drug within the dispersion can reach the distal (alveolar) regions of the lung. It has been found that certain drugs are readily absorbed through the alveolar region directly into blood circulation. Pulmonary delivery is particularly promising for the delivery of proteins and polypeptides which are difficult to deliver by other routes of administration. Such pulmonary delivery is effective both for systernic delivery and for localized delivery to treat diseases of the lungs.

Pulmonary drug delivery (including both systemic and local) can itself be achieved by different approaches, including liquid nebulizers, metered dose inhalers (MDI's) and dry powder dispersion devices. Dry powder dispersion devices are particularly promising for delivering protein and polypeptide drugs which may be readily formulated as dry powders. Many otherwise labile proteins and polypeptides may be stably stored as lyophilized or spray-dried powders by themselves or in combination with suitable powder carriers. The ability to deliver proteins and polypeptides as dry powders, however, is problematic in certain respects. The dosage of many protein and polypeptide drugs is often critical so it is necessary that any dry powder delivery system be able to accurately, and precisely (repeatedly) deliver the intended amount of drug. Moreover, many proteins and polypeptides are quite expensive, typically being many times more costly than conventional drugs on a per-dose basis. Thus, the ability to efficiently deliver the dry powders to the target region of the lung with a minimal loss of drug is critical. It is further desirable that powder agglomerates present in the dry powder be sufficiently broken up prior to inhalation by the patient to assure effective systemic absorption or other pulmonary delivery

U.S. Patent Nos. 5,785,049 and 5,740,794 describe exemplary apparatus and methods for aerosolizing a powdered medicament held within a receptacle. These apparatus and methods utilize a high pressure gas stream to extract a powdered medicament from a receptacle and to disperse the powder into the gas stream to produce an aerosol. Such apparatus and methods have proven to be generally effective in producing aerosolized medicaments for delivery to the lungs.

U.S. Patent No. 4,534,343 shows a typical metered dose inhaler where medication under pressure is stored in a canister. The drug is held in the pressurized liquid propellant that is held within the canister. The drug must be stable in this condition for the life of the system. This is undesirable for many labile medicaments such as proteins and peptides which may not be stable in this liquid for long periods of storage.

U.S. Patent No. 4,114,615 describes an aerosol inhalation device having separate propellant and medicine containers. The device comprises a valve, which acts in conjunction with a metering chamber, to supply a measured amount of propellant upon each activation of the device.

As described hereinafter, the invention provides alternative systems, devices and methods to aerosolize pharmaceutical formulations. In this way, alternative schemes are provided to assist in the delivery of pharmaceutical formulations to the lungs.

### SUMMARY OF THE INVENTION

The invention provides exemplary systems, methods and apparatus for aerosolizing pharmaceutical formulations that are typically contained within a receptacle. In a broad sense, the invention utilizes a propellant's energy to aerosolize a pharmaceutical formulation. The pharmaceutical formulation may be stored separately from the propellant until aerosolized to prolong the life of the pharmaceutical formulation. The pharmaceutical formulation may be in powder or liquid form, with the energy of the propellant being employed to place the formulation in aerosolized form so that it may be inhaled and delivered to the lungs. In one aspect, a vapor phase portion of a propellant that was previously in equilibrium with a liquid phase portion may be used to produce the aerosol.

According to one exemplary method, a metered amount of a pressurized gas is provided which was previously in equilibrium with a liquid. For example, the pressurized gas may comprise a vapor phase portion of a propellant which was previously in equilibrium with a liquid phase portion of the propellant. The metered amount of gas is released to create a high pressure gas stream. This gas stream is then flowed through an aerosolization mechanism to extract the pharmaceutical formulation from they receptacle and to disperse the pharmaceutical formulation within the gas stream to form a aerosol

The propellant utilized by the method may comprise HFCs (hydrofluorocarbon), and in particular HFAs (hydrofluoroalkanes), such as HFC 134a or HFC 227ea. Other propellants that may be used include CO₂ and CFCs (chlorofluorocarbons) 11, 12, and 114. Since CFCs have been identified as substances which deplete the stratospheric ozone layer, HFCs or HF As are preferred. The pressurized gas is preferably metered to a volume in the range from about 0.5 ml to about 2.0 ml. The pressure of the metered gas is preferably in the range from about 2.76 bar (40 psig) to about 8.27 bar (120 psig), and more preferably from about 3.45 bar (50 psig) to about 5.32 bar (80 psig). When using CO₂, the pressure of the metered gas is preferably in the range from about 55.2 bar (800 psig) to about 82.7 bar (1200 psig).

To meter the gas, the gas is preferably allowed to flow into a metering chamber. Once filled, the chamber is closed to hold the pressurized gas within the chamber. To release the pressurized gas, a valve is opened to allow the gas to rapidly escape from the metering chamber.

The invention further provides an exemplary apparatus for aerosolizing a pharmaceutical formulation held within a receptacle. The apparatus comprises a reservoir to hold a propellant having a liquid phase portion and a vapor phase portion. A metering chamber is positioned to receive and meter an amount of the vapor phase portion of the propellant. A release valve is provided to release the metered propellant to form a high pressure gas stream. The apparatus further includes an aerosolization mechanism to receive the high pressure gas stream, to extract the pharmaceutical formulation from the receptacle and to disperse the powder within the gas stream to form an aerosol.

In one aspect, a metering valve is disposed between the reservoir and the metering chamber. The metering valve is movable to an open position to allow the vapor phase portion of the propellant to enter into the metering chamber. Once the metering chamber is filled, the metering valve is preferably moved to a closed position. As one example, the metering valve may comprise a poppet valve which is pushed to move the valve to the open position. Once the chamber is filled, the poppet valve is released to allow the valve to move to the closed position.

Conveniently, a lever may be provided which is movable to close the release valve and to open the metering valve. When the lever is moved back to its starting position, the metering valve closes while the release valve remains closed. In this way, the metered propellant remains held within the metering chamber. Preferably, a fire button is provided to open the release valve and allow the metered gas to escape. In one particular aspect, a housing is provided to hold the reservoir, the metering chamber and the aerosolization mechanism. Preferably, a capture chamber is operably coupled to the housing. The capture chamber includes a mouthpiece through which the aerosolized powder may be withdrawn

In one embodiment, a system is provided for aerosolizing a pharmaceutical formulation. The system comprises a source of propellant which includes a vapor phase portion in equilibrium with a liquid phase portion. A pharmaceutical formulation is dispersed within at least a portion of the vapor phase portion of the propellant which has been separated from the liquid phase portion. Hence, with such a system a portion of a vapor propellant may be used as an energy source to aerosolize a pharmaceutical formulation

### BRIEF DESCRIPTION OF THE DRAWINGS

Fig. 1 is a schematic diagram of an exemplary system for aerosolizing a powder contained within a receptacle according to the invention.
Fig. 2 is a right side view of one embodiment of an aerosolization apparatus according to the invention.
Fig. 3 is a left side cross-sectional view of the apparatus of Fig.
Fig. 3A is a magnified view of a bottom half of the apparatus of Fig. 3
Fig. 4 is a bottom perspective view of a base unit of the apparatus of Fig. 2, with a cover being removed for convenience of illustration.
Fig. 5 is a partial left side cross-sectional view of the base unit of Fig. 4.
Fig. 6 illustrates the base unit of Fig. 4 with a lever moved to close the release valve.
Fig. 7 illustrates the base unit of Fig. 5 with the lever in the position shown in Fig. 6.
Fig. 8 illustrates the base unit of Fig. 7 with the lever moved to open a metering valve.
Fig. 9 is a top perspective view of a bottom portion of a reservoir unit of the apparatus of Fig. 3.
Fig. 10A is a bottom perspective view of a top portion of the reservoir unit.
Fig. 10B is a top perspective view of the top portion of Fig. 10A.
Fig. 11 is a top perspective view of a metering valve housing of the apparatus of Fig. 3.
Fig. 12 is a top perspective view of an actuator for the metering valve of the apparatus of Fig. 3
Fig. 13A is a side view of a left arm of the base unit of Fig. 4.
Fig. 13B is a side view of a right arm of the base unit of Fig. 4.
Figs. 14A and 14B are rear and front views, respectively, of an actuator arm for the release valve of the base unit of Fig. 4.
Fig. 15 illustrates a filling unit to refill the reservoir unit of Fig. 3.

### DETAILED DESCRIPTION OF THE SPECIFIC EMBODIMENTS

The invention provides exemplary apparatus and methods for aerosolizing pharmaceutical formulations. These formulations may be in dry powder form or they may be liquids. Examples of powdered medicaments that may be aerosolized according to the invention are described in PCT Publication No. WO96/32149. The powders aerosolized by the invention preferably have a size in the range from about 0.5 µm to about 50 µm, and more preferably in the range from about 0.5µm to about 5 µm. Alternatively, the pharmaceutical formulations may be in liquid form. The pharmaceutical formulations may be held in receptacles which have a puncturable access surface, and are sometimes referred to as "blister packages."

The invention preferably utilizes a high-pressure gas stream to extract the powder from the receptacle and to aerosolize the powder so that it may be inhaled by a patient. Examples of aerosolization mechanisms which may be utilized with the invention include those described in U.S. Patent Nos. 5,785,049 and 5,740,794, and in copending U.S. provisional patent application serial number 60/087,929, filed June 4, 1998. At least one of the specific embodiments described hereinafter utilizes an aerosolization mechanism as well as other related components which are constructed in a manner similar to those described in copending U.S. provisional patent application serial number 6,0/087,929. However, it will be appreciated that other types of aerosolization mechanisms which require the use of a high pressure gas stream to extract a powder from a receptacle and to entrain the powder within the gas stream may be utilized.

In one aspect, the invention utilizes energy from a propellant to aerosolize a pharmaceutical formulation. The propellant may include a vapor phase portion in equilibrium with a liquid phase portion. Some of the vapor phase portion may be extracted and employed as an energy source to aerosolize the pharmaceutical formulation. For example, the extracted vapor propellant may be employed to extract a powder from a receptacle, to deagglomerate the powder and to place the powder in a gas stream in aerosolized form.

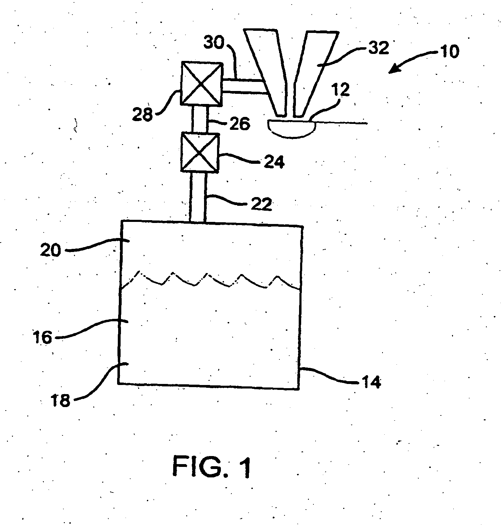

Referring now to Fig. 1, an exemplary system 10 for aerosolizing a powder contained within a receptacle 12 will be described. System 10 comprises a reservoir 14 which is filled with a propellant 16 having a liquid phase portion 18 and a vapor or gas phase portion 20. Extending from a top end of reservoir 14 is a line 22 to which is coupled a metering valve 24. Also coupled to metering valve 24 is a metering chamber 26. Disposed at the other end of metering chamber 26 is a release valve 28 to which is coupled a line 30. Line 30 extends to an aerosolization mechanism 32 which interacts with receptacle 12 to extract and aerosolize the powder contained within receptacle 12.

Propellant 16 is selected so that vapor phase portion 20 is under pressure. Hence, when metering valve 24 is opened, the pressurized propellant enters into metering chamber 26. Release valve 28 is closed to prevent the pressurized propellant from proceeding into aerosolization mechanism 32. When metering chamber 26 is filled, metering valve 24 is closed to retain a metered amount of pressurized gas within metering chamber 26. Hence, metering chamber 26 is filled with a metered amount of gas which is at a pressure determined by the type of propellant as well as the temperature of metering chamber 26.

When it is desired to aerosolize the powder, aerosolization mechanism 32 is inserted into receptacle 12 to gain access to the powder. Release valve 28 is then rapidly opened to abruptly release the metered gas within metering chamber 26. The metered gas passes through line 30 and into aerosolization mechanism 32 which extracts the powder from receptacle 12 and entrains the powder in a high pressure gas stream to form an aerosol. The aerosol may be captured in a capture chamber so that it will be available for inhalation by a patient.

Hence, system 10 is configured to operate simply by releasing a metered amount of pressurized gas which was obtained by extracting a vapor phase portion of a propellant that was previously in equilibrium with a liquid phase portion. System 10 is configured to meter a fixed volume of the gas at a fixed pressure. In this way, aerosolization mechanism 32 is able to consistently receive a known volume of gas which is at a known pressure so that it may optimally extract and aerosolize the powder within receptacle 12. With such a configuration, a variety of advantages are provided. For example, system 10 is easy to operate because it only requires the opening of metering valve 24 when metering the gas, and the opening of release valve 28 to aerosolize the powder. Another advantage is that the size of system 10 may be reduced by utilizing a propellant. Typically, only a small volume of propellant is needed so that the size of reservoir 14 may be made relatively small. Such a configuration reduces the overall size of system 10 so that it will be easy and convenient for a user to carry and store. Typically, system 10 will have a longer life than comparable MDI devices because system 10 employs only a vapor phase portion of the propellant to aerosolize the powder rather than the liquid phase as with MDI devices. Further, system 10 is more simple to manufacture and easier to use (because the patient is not required to shake the propellant) than comparable MDI devices since it does not require a drug to be suspended within the liquid phase portion of the propellant.

Preferable propellants that may be utilized with system 10 include HFA or HFC, such as hydrofluorocarbon 134a (HFC-134a, 1,1,2-tetrafluoroethane) and hydrofluorocarbon 227ea (HFC-227ea, 1,1,1,2,3,3,3-heptafluoropropane). These propellants are known in the art and are commercially available from the Minnesota Mining & Manufacturing Co. and Dupont. Such propellants are particularly useful in that they do not utilize CFCs. Other propellants that may be used with the invention include CO₂, CFCs, and the like.

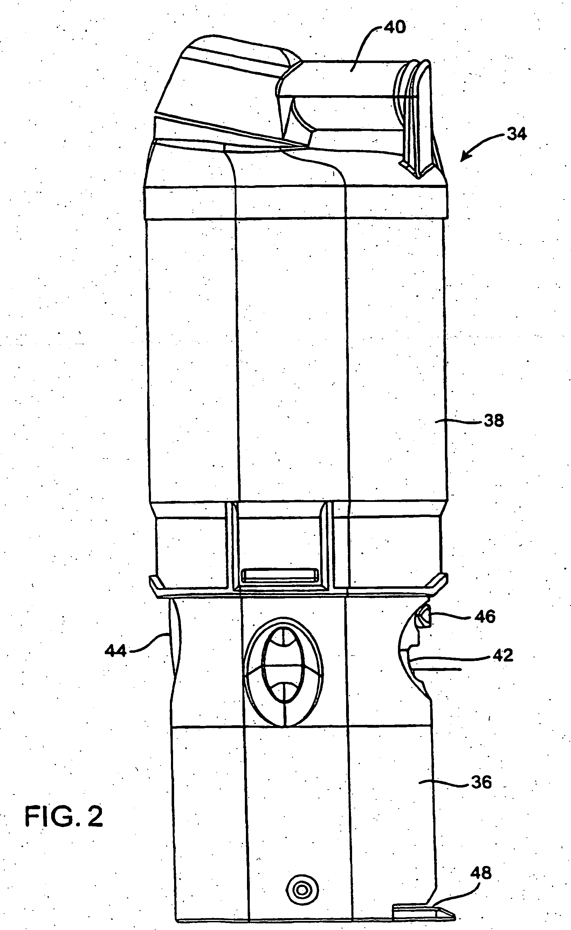

Referring now to Fig. 2, an exemplary embodiment of an apparatus 34 for aerosolizing a powder medicament will be described. Apparatus 34 comprises a base unit 36 and a capture chamber 38 which is removably attached to base unit 36. Capture chamber 38 is configured to slide over base unit 36 to reduce the overall size of apparatus 34 during storage and to provide a degree of protection to the components within base unit 36. Capture chamber 38 also includes a mouthpiece 40 that is rotatable between an open position and a closed position. During aerosolization, mouthpiece 40 is in the closed position as illustrated in Fig. 2. When the patient is ready to inhale the aerosolized medicament, mouthpiece 40 is rotated 180 degrees to the open position where the patient may place his mouth over the mouthpiece and inhale the powdered medicament from capture chamber 38.

While their size is smaller, the overall shape and appearance of capture chamber 38 and base unit 36 are similar to those described in copending application number 60/087,929. By utilizing a propellant to aerosolize a medicament, base unit 36 does not require a cylinder to compress air from a large volume and does not require a lever which fits the patient's hand to provide the energy for compressing the gas. Hence, base unit 36 may have its size reduced. Merely by way of example, base unit 36 may have a height that is in the range from about 8 cm to 12 cm. Capture chamber 38 may have a height in the range from about 9 cm to about 13 cm.

Still referring to Fig. 2, base unit 36 includes an opening 42 into which a receptacle containing a powdered medicament is inserted. Base unit 36 also includes a fire button 44 which is pressed to pierce the receptacle and to release a volume of pressurized gas to aerosolize the medicament in the receptacle as described hereinafter. A release button 46 is provided to allow the receptacle to be removed from opening 42 after the medicament has been aerosolized. Base unit 36 further includes a lever 48 which may be operated to meter an amount of pressurized gas as described hereinafter.

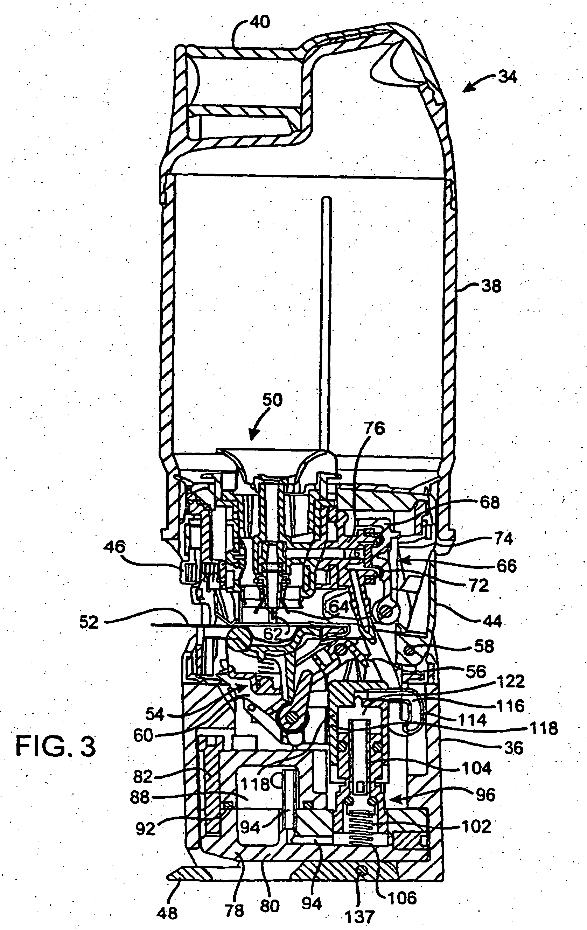

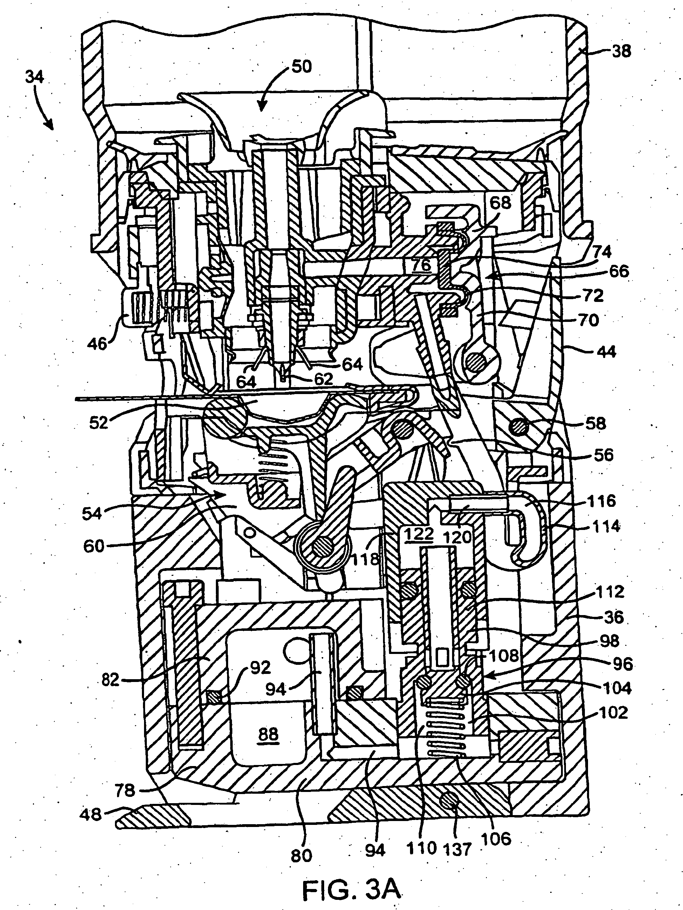

Referring now to Figs. 3 and 3A, the components within base unit 36 will be described. Disposed within base unit 36 is an aerosolization mechanism 50 which is employed to extract a medicament from a receptacle 52, to entrain the medicament within a high pressure gas stream, and to deliver the aerosolized medicament into capture chamber 38. Base unit 36 also includes a carriage assembly 54 which moves receptacle 52 to engage aerosolization mechanism 50 when fire button 44 is pressed. Aerosolization mechanism 50, carriage assembly 54, and their related components are constructed essentially identical to those described in copending application number 60/087,929. As such, their manner of construction will only be briefly described in this application.

To operate apparatus 34, receptacle 52 is inserted into opening 42 (see Fig. 2) as previously described. Fire button 44 is then pushed to engage receptacle 52 with aerosolization mechanism 50. More specifically, when fire button 44 is pushed, gear teeth 56 are pivoted about a pivot pin 58, causing a lifter 60 of carriage assembly 54 to move receptacle 52 toward aerosolization mechanism 50. When fire button 44 is fully depressed, a pointed tip 62 and side punches 64 pierce through receptacle 52 and enter into the receptacle. Depression of fire button 44 also operates a valve 66 to allow for the release of a metered volume of high pressure gas into aerosolization mechanism 50 so that the powder within receptacle 52 may be aerosolized. More specifically, repression of fire button 44 causes a valve actuator 68 of an actuator arm 70 to be released from an over center position, thereby unlocking valve 66.

Once valve 66 is unlocked, the metered amount of high pressure gas within base unit 36 causes valve 66 to "pop open." More specifically, the release of valve actuator 68 causes the metered amount of high pressurized gas to come into contact with the underside of the diaphragm 72 causing a valve seat 74 to be lifted from a passage 76. In this manner, the propellant is allowed to flow through passage 76 and into aerosolization mechanism 50. The high pressurized gas then extracts the powdered medicament from receptacle 52, deagglomerates the powdered medicament and disperses the powdered medicament into capture chamber 38 as previously described. Hence, to operate apparatus 34, the user simply inserts receptacle 52 and then presses fire button 44 which will cause the aerosolized medicament to be introduced into capture chamber 38 where it may be withdrawn through mouthpiece 40.

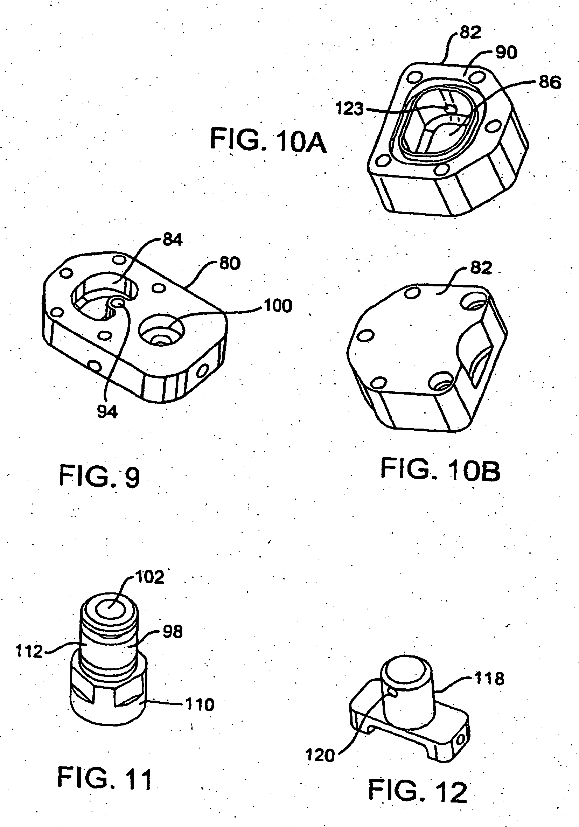

Still referring to Figs. 3 and 3A, the manner in which the pressurized gas which is supplied to aerosolization mechanism 50 through valve 66 will be described. Base unit 36 includes a reservoir unit 78 which is constructed of a bottom member 80 and a top member 82. Bottom member 80 is also shown in Fig: 9, and top member 82 is shown in Figs. 10A and 10B. Bottom member 80 includes an opening 84 and top member 82 includes an opening 86. When top member 82 is secured to bottom member 80, openings 84 and 86 together form a cavity 88 which holds the propellant. Conveniently, top member 82 includes groove 90 for holding an O-ring seal 92 so that the propellant does not escape from cavity 88. Traveling through bottom member 80 is a passage 94. Cavity 88 is configured such that it may hold a liquid phase portion of the propellant in equilibrium with a vapor phase portion. The vapor phase portion of the propellant extends into passage 94 where it is prevented from escaping by a mitering valve 96

As also shown in Fig. 11, metering valve 96 includes a housing 98 which is received into an opening 100 of bottom member 80 (see Fig. 9). Housing 98 has a central passage 102 which communicates with passage 94 of bottom member 80. As best shown in Figs. 3 and 3A, a valve poppet 104 is positioned within passage 102. A spring 106 biases valve poppet 104 upward so that an O-ring 108 disposed about valve poppet 104 engages housing 98. In this way, metering valve 96 is configured to be in a normally closed position where the vapor phase propellant is prevented from moving beyond valve poppet 104.

Housing 98 may conveniently be divided into a bottom portion 110 and as top portion 112. When metering valve 96 is in the closed position, the vapor phase propellant remains within bottom portion 110. When metering valve 96 is opened, the vapor phase propellant rushes into top portion 112 and into a metering tube 114. As such, the volume within top portion 112 and within metering tube 114 define a metering chamber 116. At the opposite end of metering chamber 116 is valve 66 whose function is to prevent the escape of the pressurized gas from metering chamber 116 until valve actuator 68 is released as previously described. When valve 66 is opened, the gas within metering chamber 116 rushes into passage 76 where it enters into aerosolization mechanism 50.

As best shown in Figs. 3, 4 and 12, a valve actuator 118 is disposed over housing 98. Valve actuator 118 has a passage 120 which allows for the passage of gases between an interior 122 and metering tube 114. As best shown in Figs. 3 and 3A, valve poppet 104 extends vertically above housing 98. In this way, when valve actuator 118 is moved downward, it will eventually engage valve poppet 104 to cause spring 106 to compress and to move O-ring 108 from housing 98. In this way, pressurized gas within bottom portion 110 may escape into metering chamber 116.

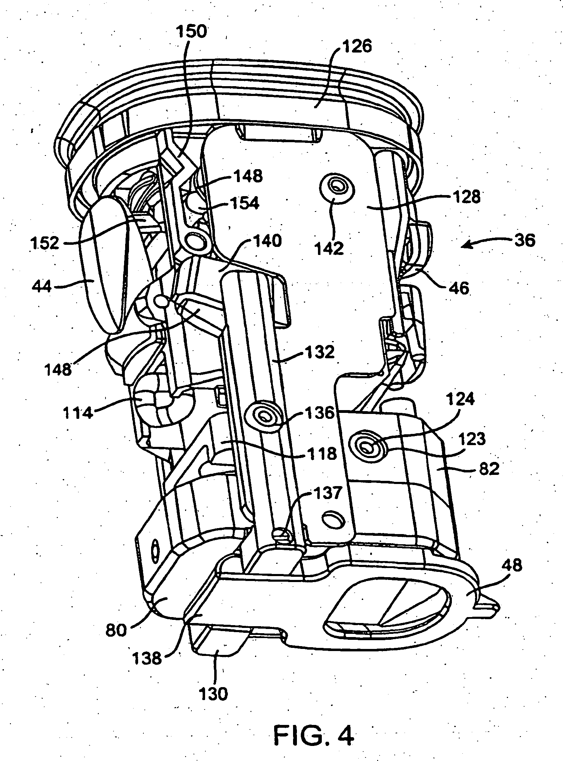

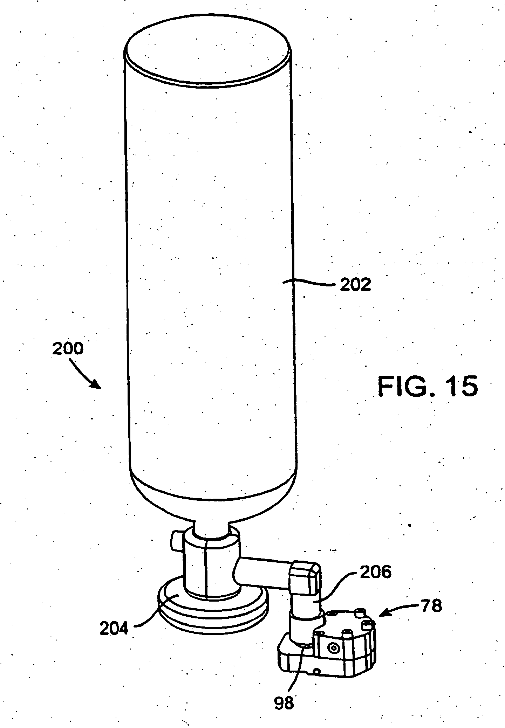

As best shown in Figs. 4 and 10A, top member 82 includes an opening 123 through which a bleed valve 124 is inserted. Bleed valve 124 may conveniently be constructed as a screw having an O-ring. Bleed valve 124 is opened when filling the cavity 88 with a liquid propellant. Preferably, cavity 88 is filled by inserting the propellant through passage 102 of housing 98 as described in greater detail with reference to Fig. 15.

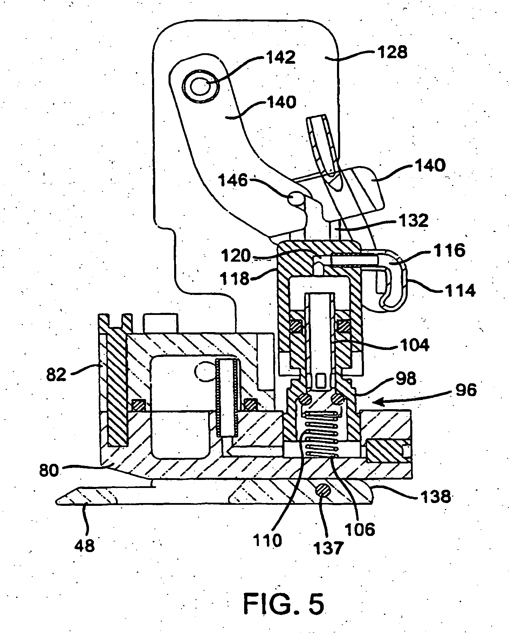

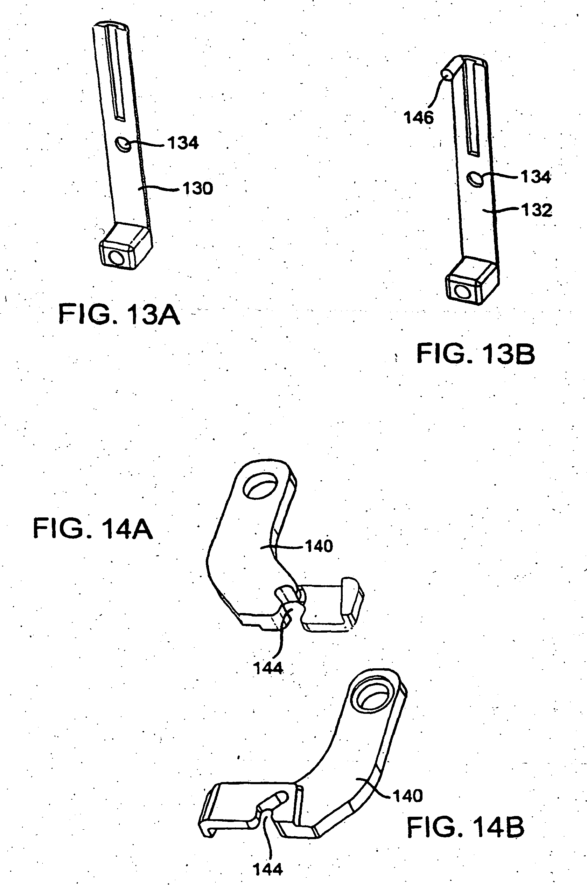

Referring now to Figs. 4 and 5, construction of base unit 36 to facilitate opening and closing of valves 66 and 96 (See Fig. 3A) will be described. As best shown in Fig. 4, base unit 36 includes a chassis 126 to which a frame 128 is coupled. Although not shown, a similar frame is also coupled to chassis 126 on the other side of base unit 36. Movably coupled to frames 128 are a left arm 130 and a right arm 132. Arms 130 and 132 are also shown in Figs. 13A and 13B, respectively. Arms 130 and 132 include a hole 134 through which a pin 136 is inserted as shown in Fig. 4. Pins 136 extend through slots (not shown) in frames 128 so that arms 130 and 132 may be moved vertically up and down relative to frames 128. Lever 48 is pivotally coupled between arms 130 and 132 by a pin 137. Lever 48 includes an extension 138 which engages bottom member 80 when lever 48 is pivoted. In so doing, lever 48 is moved away from bottom member 80 to move arms 130 and 132 vertically downward relative to frames 128.

Also pivotally coupled to frame 128 is an actuator arm 140. As best shown in Fig. 4, a pin 142 is employed to pivotally couple actuator arm 140 to frame 128. Actuator arm 140 is also shown in Figs. 14A and 14B. As best shown in Figs. 14A and 14B, actuator arm 140 includes a slot 144 through which a pin 146 (see Fig. 13B) on right arm 132 moves when right arm 132 is moved relative to frame 128. In this way, movement of arm 132 relative to frame 128 causes actuator arm 140 to pivot about pin 142.

As best shown in Fig. 4, actuator arm 140 engages a release valve set arm 148. Valve set arm 148 is configured to pivot with actuator arm 140. Further, valve set arm 148 is employed to move actuator arm 70 (see Fig. 3A) so that valve 66 may be held in a closed position until valve set arm 148 is moved to allow valve 66 to open. Conveniently, valve set arm 148 and actuator arm 70 may be configured to be essentially identical to similar components described in copending application serial number 60/087,929.

Chassis 126 includes a boss 150 under which valve set arm 148 may be positioned to maintain valve 66 in the closed position. Valve set arm 148 is moved under boss 150 by moving lever 48, which causes valve set arm 148 to pivot until received under boss 150. At this position, valve 66 is in an over center position where it will remain closed until valve set arm 148 is moved out from under boss 150.

Fire button 44 includes a tab 152 which engages a post 154 on valve set arm 148 when fire button 44 is depressed. As tab 152 pushes valve set arm 148 out from under boss 150, valve actuator arm 70 (see Fig. 3A) is allowed to move back away from its over center position, unclamping diaphragm 72. In this way, the pressurized gas within metering chamber 116 is allowed to unseat valve actuator 68 and rush into passage 76 as previously described

As best shown in Fig. 4, pin 136 also couples right arm 132 to valve actuator 118. Left arm 130 is coupled to valve actuator 118 in a similar manner. In this way, when lever 48 is moved, valve actuator 118 is moved downward to open valve 96 (see Fig. 3A). Hence, operation of lever 48 closes valve 66 and subsequently opens metering valve 96. In this way, the metering chamber may be filled with pressurized gas. As lever 48 is moved back, metering valve 96 is closed so that the pressurized gas remains held within the metering chamber.

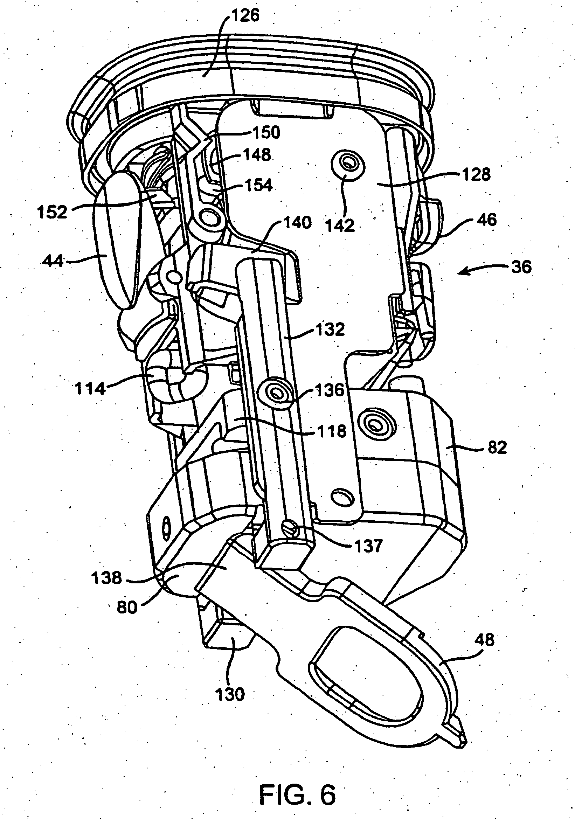

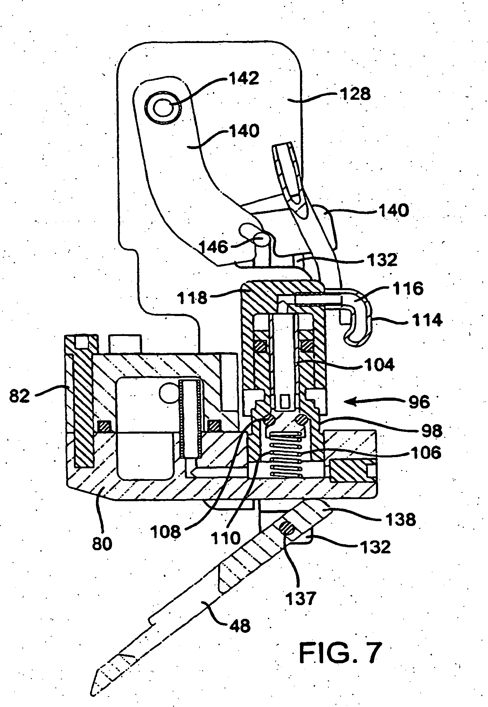

As shown in Figs. 4 and 5, base unit 36 has lever 48 in a home position. As previously described, in this position metering valve 96 is in its normally closed position. Assuming that fire button '44 was previously pressed, valve 66 is in its normally open position. As shown in Figs. 6 and 7, lever 48 is beginning to be moved away from its home position. In so doing, extension 138 engages bottom member 80 to move arms 130 and 132 downward. In turn, valve set arm 148 is moved underneath boss 150 to close valve 66. At the same time, valve actuator 118 is moved downward until it engages valve poppet 104.(see Fig. 7). As shown in Fig. 5, valve actuator 118 is spaced apart from valve poppet 104 when lever 48 is in home position. Hence, when reaching the position shown in Fig. 7, actuator 118 has not yet pushed valve poppet 104 downward. As such, valve 66 is closed before metering valve 96 is opened.

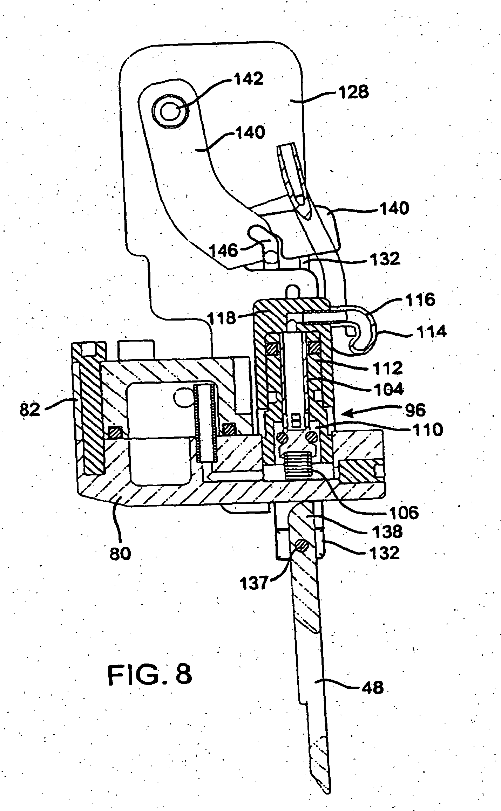

As shown in Fig. 8, lever 48 is fully pivoted, causing valve actuator 118 to force valve poppet 104 downward. In so doing, metering valve 96 is opened to allow the pressurized gas within bottom portion 110 to escape into metering chamber 116. Lever 48 may then be moved back to the home position to close metering valve 96. As lever 48 is moved back to the home position, valve set arm 148 remains under boss 150 so that the pressurized gas is held within metering chamber 116. Fire button 44 may then be depressed to open valve 66 to allow the powdered medicament to be aerosolized as previously described, Although not shown, an interlock may be provided to ensure that fire button 44 may not be actuated unless a receptacle has been placed in apparatus 34. In this way, the propellant is prevented from being released unless a receptacle is inverted.

One advantage of utilizing reservoir unit 78 to hold a propellant is that the overall size of base unit 36 may be reduced. At the same time, reservoir unit 78 is able to hold a sufficient volume of propellant so that many doses may be supplied to the patient. Merely by way of example, cavity 88 may be configured to hold about 2cc of HFC liquid. This will allow apparatus 34 to be actuated approximately 100 times using about 0.65cc of vapor per actuation. As such, metering chamber 116 will preferably be configured to hold about 0.65cc of the HFC vapor. However, it will appreciated that reservoir unit 78 and metering chamber 116 may be configured to hold other volumes of propellant.

Although shown with reservoir unit 78, it will be appreciated that alternative embodiments of propellant sources may be provided. For example, reservoir unit 78 may be replaced with a canister or other replaceable tank to facilitate easy replacement of the canister or tank when the propellant has been depleted. Because it is desirable to periodically replace aerosolization mechanism 50, the source of propellant may be configured to be coupled to the aerosolization mechanism so that both can be removed and replaced once the propellant has been depleted.

When utilizing HFA as the propellant, the pressure of the vapor phase portion held within metering chamber 116 is preferably in the range from about 40 psi to about 120 psi, and more preferably about 50 psi to about 80 psi. Configuring apparatus 34 to operate utilizing a wide range of pressures is advantageous in that the pressure of the HFC may vary depending upon the ambient temperature. As such, apparatus 34 may be used in a wide variety of environments and climate.

Fig. 15 illustrates a filling unit 200 that may be employed to fill reservoir unit 78 (see Fig. 3) with a propellant. Filling unit 200 comprises a supply tank 202 to store the propellant. Coupled to tank 202 is a valve 204 that may be opened to allow the propellant to be transferred from tank 202. Tubing 206 extends from valve 204 and is configured to fit over metering valve housing 98 (see also Fig. 11).

In use, tubing 206 is placed over housing 98 and valve 204 is opened. The propellant passes through housing 98 and into cavity 88 (see Fig. 10A). Bleed valve 124 may be opened to provide a vent.

Optionally, apparatus 34 may include a visual display of the number of times the apparatus has been used and/or the number of times it may be used before requiring a propellant refill and/or replacement. Conveniently, a counting system may be coupled to the fire button 44 or lever 48 to count the number of actuations.

The invention has now been described in detail for purposes of clarity of understanding. However, it will be, appreciated that certain changes and modifications may be practiced within the scope of the appended claims.

## Claims

1. A method for aerosolizing a pharmaceutical formulation contained within a receptacle (12), comprising:
providing a metered amount of a pressurized gas that was previously in equilibrium with a liquid;
releasing the metered gas to create a high pressure gas stream; and flowing the high pressure gas stream through an aerosolization mechanism (32) which in operation, by actuating a fire button (44), is brought into engagement with the receptacle (12) and is adapted to receive the high pressure gas stream such that the high pressure gas stream flows past the receptacle thereby extracting the pharmaceutical formulation by drawing it from the receptacle (12) and dispersing the pharmaceutical formulation within the gas stream to form an aerosol, wherein the pharmaceutical formulation is not administered to a human or animal body.

2. A method as in claim 1, wherein the pressurized gas comprises HFC.

3. A method as in claim 1 or 2, wherein the pharmaceutical formulation is in dry powder form.

4. A method as in any of claims 1 to 3, further comprising opening a valve (28) to release the pressurized gas.

5. An apparatus for aerosolizing a pharmaceutical formulation held within a receptacle (12), the apparatus comprising:
a reservoir (14) which is adapter to hold a propellant (16) having in equilibrium a liquid phase portion and a vapor phase portion;
a metering chamber (36) which is adapted to receive an an amount of the vapor phase portion of the propellant (16);
a release valve (28) which is adapted to release the metered propellant (16) to form a high pressure gas stream; a fire button (44) and
an aerosolization mechanism (32) which in operation, by actuating said fire button (44), is brought into engagement with the receptacle (12) and is adapted to receive the high pressure gas stream such that the high pressure gas stream flows past the receptacle thereby extracting the pharmaceutical formulation by drawing it from the receptacle (12) and to disperse the pharmaceutical formulation within the gas stream to form an aerosol.

6. An apparatus as in claim 5, further comprising a metering valve (24) disposed between the reservoir (14) and the metering chamber (26), wherein the metering valve (24) is movable to an open position to allow the vapor phase portion of the propellant to enter the metering chamber (26).

7. An apparatus as in claim 6, further comprising a lever which is moveable to close the release valve (28) and to open the metering valve (24).

8. An apparatus as in any of claims 5 to 7, wherein said fire button (44) is adapted to open the release valve.

9. An apparatus as in any of claims 5 to 8, further comprising a housing to hold the reservoir (14), the metering chamber (26) and the aerosolization mechanism (32).

10. An apparatus as in claim 9, further comprising a capture chamber operably coupled to the housing, wherein the capture chamber includes a mouthpiece.

11. An apparatus as in claim 6, wherein the metering valve (24) comprises a poppet valve which is pushed to move the valve to the open position.

## Patentansprüche

1. Verfahren zum Aerosolisieren einer in einem Behälter (12) enthaltenen pharmazeutischen Formulierung, mit:
Bereitstellen einer dosierten Menge eines unter Druck stehenden Gases, das zuvor mit einer Flüssigkeit im Gleichgewicht war;
Freisetzen des dosierten Gases, um einen Gasstrom hohen Drucks zu erzeugen; und
Durchleiten des Gasstroms hohen Drucks durch einen Aerosolisierungsmechanismus (32), der in Betrieb durch Betätigung eines Auslöseknopfs (44) in Eingriff mit dem Behälter (12) gebracht wird und geeignet ist, den Gasstrom hohen Drucks zu empfangen, so daß der Gasstrom hohen Drucks an dem Behälter vorbeiströmt und dabei die pharmazeutische Formulierung extrahiert, indem er die pharmazeutische Formulierung aus dem Behälter (12) zieht und in dem Gasstrom verteilt, um ein Aerosol zu bilden, wobei die pharmazeutische Formulierung nicht an einen menschlichen oder tierischen Körper verabreicht wird.

2. Verfahren nach Anspruch 1, wobei das unter Druck stehende Gas HFC aufweist.

3. Verfahren nach Anspruch 1 oder 2, wobei die pharmazeutische Formulierung in Form eines Trockenpulvers ist.

4. Verfahren nach einem der Ansprüche 1 bis 3, ferner mit Öffnen eines Ventils (28), um das unter Druck stehende Gas freizusetzen.

5. Vorrichtung zum Aerosolisieren einer in einem Behälter (12) enthaltenen pharmazeutischen Formulierung, wobei die Vorrichtung aufweist:
ein Reservoir (14), das in der Lage ist, ein Treibmittel (16), das einen flüssigen Phasenteil und einen Dampfphasenteil im Gleichgewicht hat, zu halten;
eine Dosierkammer (26), die in der Lage ist, eine Menge des Dampfphasenteils des Treibmittels (16) zu empfangen;
ein Freisetzungsventil (28), das in der Lage ist, das dosierte Treibmittel (16) freizusetzen, um einen Gasstrom hohen Drucks zu erzeugen;
einen Auslöseknopf (44); und
einen Aerosolisierungsmechanismus (32), der in Betrieb durch Betätigung des Auslöseknopfs (44) in Eingriff mit dem Behälter (12) gebracht wird und in der Lage ist, den Gasstrom hohen Drucks zu empfangen, so daß der Gasstrom hohen Drucks an dem Behälter vorbeiströmt und dabei die pharmazeutische Formulierung extrahiert, indem er die pharmazeutische Formulierung aus dem Behälter (12) zieht und in dem Gasstrom verteilt, um ein Aerosol zu erzeugen.

6. Vorrichtung nach Anspruch 5, ferner mit einem zwischen dem Reservoir (14) und der Dosierkammer (26) angeordneten Dosierventil (24), das zu einer offenen Position bewegbar ist, um dem Dampfphasenteil des Treibmittels den Eintritt in die Dosierkammer (26) zu ermöglichen.

7. Vorrichtung nach Anspruch 6, ferner mit einem Hebel, der bewegbar ist, um das Freisetzungsventil (28) zu schließen und das Dosierventil (24) zu öffnen.

8. Vorrichtung nach einem der Ansprüche 5 bis 7, wobei der Auslöseknopf (44) in der Lage ist, das Freisetzungsventil zu öffnen.

9. Vorrichtung nach einem der Ansprüche 5 bis 8, ferner mit einem Gehäuse, um das Reservoir (14), die Dosierkammer (26) und den Aerosolisierungsmechanimus (32) zu halten.

10. Vorrichtung nach Anspruch 9, ferner mit einer Auffangkammer, die in Betrieb mit dem Gehäuse verbunden ist, wobei die Auffangkammer ein Mundstück aufweist.

11. Vorrichtung nach Anspruch 6, wobei das Dosierventil (24) ein Tellerventil aufweist, das gestoßen wird, um das Ventil in die offene Position zu bewegen.

## Revendications

1. Procédé pour pulvériser une formulation pharmaceutique contenue dans un récipient (12), comprenant les étapes consistant à :
■ prévoir une quantité dosée d'un gaz sous pression qui était au préalable en équilibre avec un liquide ;
■ libérer le gaz dosé pour créer un flux de gaz à haute pression ; et
■ faire écouler le flux de gaz à haute pression par un mécanisme de pulvérisation (32) qui en fonctionnement, en actionnant un petit bouton (44), est amené en mise en prise avec le récipient (12) et est adapté pour recevoir le flux de gaz à haute pression de sorte que le flux de gaz à haute pression s'écoule au-delà du récipient, faisant ainsi sortir la formulation pharmaceutique en l'aspirant du récipient (12) et en dispersant la formulation pharmaceutique dans le flux de gaz pour former un aérosol, dans lequel la formulation pharmaceutique n'est pas administrée à un corps humain ou animal.

2. Procédé selon la revendication 1, dans lequel le gaz sous pression comprend du HFC.

3. Procédé selon la revendication 1 ou 2, dans lequel la formulation pharmaceutique se présente sous forme de poudre sèche.

4. Procédé selon l'une quelconque des revendications 1 à 3, comprenant en outre l'étape consistant à ouvrir une soupape (28) pour libérer le gaz sous pression.

5. Appareil pour pulvériser une formulation pharmaceutique contenue dans un récipient (12), l'appareil comprenant :
■ un réservoir (14) qui est adapté pour contenir un gaz propulseur (16) ayant, en équilibre, une partie en phase liquide et une partie en phase vapeur ;
■ une chambre de dosage (26) qui est adaptée pour recevoir une quantité de la partie en phase vapeur du gaz propulseur (16) ;
■ une soupape de libération (28) qui est adaptée pour libérer le gaz propulseur dosé (16) pour former un flux de gaz à haute pression ;
■ un petit bouton (44) ; et
■ un mécanisme de pulvérisation (32) qui en fonctionnement, en actionnant ledit petit bouton (44), est amené en mise en prise avec le récipient (12) et est adapté pour recevoir le flux de gaz à haute pression de sorte que le flux de gaz à haute pression s'écoule au-delà du récipient, faisant ainsi sortir la formulation pharmaceutique en l'aspirant du récipient (12) et pour disperser la formulation pharmaceutique dans le flux de gaz pour former un aérosol.

6. Appareil selon la revendication 5, comprenant en outre une soupape de dosage (24) disposée entre le réservoir (14) et la chambre de dosage (26), dans lequel la soupape de dosage (24) est déplaçable en position ouverte pour permettre à la partie en phase vapeur du gaz propulseur d'entrer dans la chambre de dosage (26).

7. Appareil selon la revendication 6, comprenant en outre un levier qui est déplaçable pour fermer la soupape de libération (28) et pour ouvrir la soupape de dosage (24).

8. Appareil selon l'une quelconque des revendications 5 à 7, dans lequel ledit petit bouton (44) est adapté pour ouvrir la soupape de libération.

9. Appareil selon l'une quelconque des revendications 5 à 8, comprenant en outre un boîtier pour contenir le réservoir (14), la chambre de dosage (26) et le mécanisme de pulvérisation (32).

10. Appareil selon la revendication 9, comprenant en outre une chambre de capture couplée de manière opérationnelle au boîtier, dans lequel la chambre de capture comprend un embout buccal.

11. Appareil selon la revendication 6, dans lequel la soupape de dosage (24) comprend une soupape à clapet qui est poussée pour déplacer la soupape dans la position ouverte.
